# FASCICULE DE BREVET EUROPEEN

(11) **EP 0 685 224 B1**
(45) Date de publication et mention de la délivrance du brevet: **30.06.1999**
(21) Numéro de dépôt: 95401058.3
(22) Date de dépôt: 05.05.1995
(51) Int. Cl.: A61K 7/42

(54) **Compositions cosmétiques photoprotectrices à base d'un mélange synergétique de filtres et de nanopigments et utilisations**
Kosmetische Sonnenschutzmittel enthaltend eine synergistische Mischung aus Filtern und Nanopigmenten und Verwendungen
Cosmetic sunscreening compositions containing a synergic mixture of filters and nanopigments and uses

(30) Priorité: 03.06.1994 FR 9406832
(43) Date de publication de la demande: 06.12.1995
(73) Titulaire: L'OREAL, 75008 Paris (FR)
(72) Inventeur: Ascione, Jean-Marc, F-75010 Paris (FR); Allard, Delphine, F-92700 Colombes (FR)
(74) Mandataire: Andral, Christophe André Louis

(56) Documents cités:
- EP-A- 0 457 687
- EP-A- 0 518 772

## Description

La présente invention concerne de nouvelles compositions cosmétiques améliorées à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable et de préférence de type émulsion huile-dans-eau, une association entre (i) un système organique filtrant constitué de deux filtres particuliers et convenablement sélectionnés, à savoir d'une part la 2,4,6-tris[p-(2'-éthylhexyl-1'-oxycarbonyl)anilino]-1,3,5-triazine et, d'autre part, l'acide benzène 1,4-di(3-méthylidène-10-camphosulfonique), et (ii) à titre d'agents photoprotecteurs agissant par blocage physique du rayonnement (réflecteurs et/ou diffuseurs d'UV), des nanopigments minéraux à base d'oxydes métalliques, et en particulier d'oxyde de titane, cette association conférant auxdites compositions, par un effet de synergie, des facteurs de protection solaire améliorés.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber selectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Ainsi, dans la demande de brevet EP-A- 0 457 687, il a déja été proposé un système filtrant particulièrement intéressant constitué par l'association entre un filtre UV-A hydrophile, à savoir l'acide benzène 1,4-di(3-méthylidène-10-camphosulfonique), et un filtre UV-B lipophile, à savoir la 2,4,6-tris[p-(2'-éthylhexyl-1'-oxycarbonyl)anilino]-1,3,5-triazine, cette association présentant pour avantages principaux de conduire à des compositions antisolaires non toxiques qui d'une part, par un effet de synergie entre lesdits deux filtres, possèdent des indices de protection élevés, et ceci dans une large gamme de longueurs d'onde UV (280-400 nm), qui, d'autre part, sont stables thermiquement et photochimiquement, et qui, enfin, présentent de bonnes propriétés cosmétiques. Toutefois, les indices de protection sur peau conférés par les compositions décrites dans ce document peuvent, et en particulier pour les peaux dites très sensibles et/ou continuellement exposées au soleil, apparaitre comme encore insuffisants, et il serait donc utile, à cet égard, de pouvoir encore améliorer les propriétés photoprotectrices de ces compositions de l'art antérieur.

La présente invention vise à la satisfaction d'un tel besoin.

Ainsi, à la suite d'importantes recherches menées dans le domaine de la photoprotection évoqué ci-dessus, la Demanderesse a pu mettre en évidence l'existence d'un nouvel effet de synergie capable d'apparaître, de façon inattendue et surprenante, dans le cas particulier où les deux filtres solaires susmentionnés viennent à être associés à des nanopigments, cet effet permettant d'obtenir des compositions antisolaires présentant des indices de protection nettement améliorés, et en tous cas supérieurs à ceux qui peuvent être obtenus, à concentration égale de filtres ou de nanopigments et à nature identique de support, avec les filtres utilisés seuls ou avec les nanopigments utilisés également seuls. Par ailleurs, il a été constaté que l'introduction desdits nanopigments dans le sytème filtrant constitué par la combinaison connue des deux filtres précités ne perturbait ou ne modifiait en rien les autres propriétés avantageuses attachées intrinsèquement à cette combinaison. Grâce à l'invention, on peut donc obtenir finalement des compositions antisolaires cosmétiquement acceptables, non toxiques, offrant une photoprotection large (UV-A + UV-B) et très élevée, et ceci avec des concentrations en filtres limitées.

Ces découvertes sont à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (i) un système organique filtrant constitué par l'association d'un premier filtre qui est la 2,4,6-tris[p-(2'-éthylhexyl-1'-oxycarbonyl)anilino]-1,3,5-triazine avec un deuxième filtre qui est l'acide benzène 1,4-di(3-méthylidène-10-camphosulfonique), ce deuxième filtre pouvant éventuellement être sous une forme partiellement ou totalement neutralisée, et (ii) des nanopigments minéraux à base d'oxydes métalliques.

Au sens de la présente invention, on entend par "nanopigments" des pigments dont la taille moyenne des particules primaires n'excède pas 100 nm, cette taille étant de préférence comprise entre 5 nm et 100 nm, et plus préférentiellement encore comprise entre 10 et 50 nm.

Bien que l'utilisation de pigments minéraux à base d'oxydes métalliques, et en particulier d'oxyde de titane, soit, dans le domaine de la protection solaire, connue d'une manière générale, et que l'on sache en particulier que ces substances, qu'elles soient ou non associées avec des filtres organiques usuels absorbeurs d'UV-A et/ou UV-B, sont capables d'apporter aux compositions antisolaires qui les contiennent un certain pouvoir photoprotecteur propre ou complémentaire relativement limité, et ceci en agissant par blocage physique des rayons UV (réflection et/ou diffusion), il reste toutefois que les propriétés potentialisantes ou synergétiques des pigments particuliers (nanopigments) qui sont utilisés dans le cadre de la présente invention à l'égard du pouvoir photoprotecteur de l'association spécifique filtrante {Acide benzène 1,4-di(3-méthylidène-10-camphosulfonique) + 2,4,6-tris[p-(2'-éthylhexyl-1'-oxycarbonyl) anilino]-1,3,5-triazine} n'ont, quant à elles, jamais été décrites ou même suggérées. Par ailleurs, et comme le montreront les exemples donnés ci-après, il est à noter que la mise en oeuvre de pigments autres que ceux conformes à la présente l'invention (nanopigments) ne conduit à aucun effet de synergie avec l'association filtrante précitée.

La présente invention a également pour objet l'utilisation des compositions ci-dessus comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaitront à la lecture de la description détaillée qui va suivre.

La 2,4,6-tris[p-(2'-éthylhexyl-1'-oxycarbonyl)anilino]-1,3,5-triazine (premier filtre) mise en ouvre dans le cadre de la présente invention est un filtre connu en soi (voir le document EP-A- 0 457 687 précité), actif dans l'UV-B et se présentant sous une forme solide, qui est vendu notamment sous la dénomination commerciale de "UVINUL T 150" par la Société BASF. Ce produit répond à la formule (I) suivante : 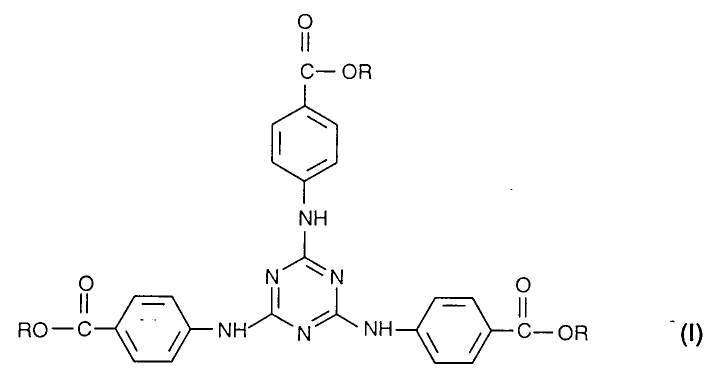 dans laquelle R désigne un radical 2-éthyl hexyle.

L' acide benzène 1,4-di(3-méthylidène-10-camphosulfonique) et ses différents sels (deuxième filtre), décrits notamment dans les demandes de brevets FR-A- 2 528 420 et FR-A- 2 639 347, sont des filtres eux-aussi déja connus en soi (filtres dits à large bande) capables en effet d'absorber les rayons ultraviolets de longueur d'ondes comprises entre 280 et 400 nm, avec des maxima d'absorption compris entre 320 et 400 nm, en particulier aux alentours de 345 nm. Ces filtres répondent à la formule générale (II) suivante : 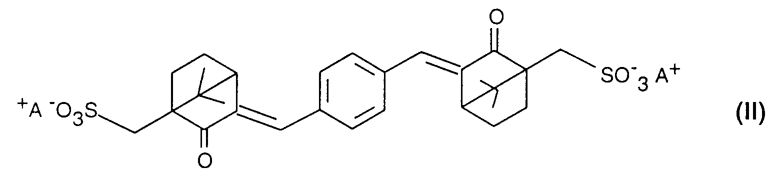 dans laquelle A désigne un atome d'hydrogène, un métal alcalin ou encore un radical NH(R)₃⁺ dans lequel les radicaux R, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺. Il est bien entendu que les composés de formule (I) ci-dessus peuvent donner lieu à l'isomère "cis-trans" autour d'une ou plusieurs double(s) liaison(s) et que tous les isomères rentrent dans le cadre de la présente invention.

Le premier filtre peut être présent dans les compositions selon l'invention à une concentration comprise entre 0,1 et 10 % en poids, et de préférence entre 0,5 et 5 % en poids, par rapport au poids total de la composition et le deuxième filtre peut être, quant à lui, présent à des teneurs comprises entre 0,2 et 15 % en poids, et de préférence entre 0,5 et 10 % en poids, toujours par rapport au poids total de la composition de préférence, la teneur globale du mélange entre ces deux filtres n'excède pas 15 % du poids total de la composition finale.

Les oxydes métalliques constitutifs des nanopigments utilisables dans le cadre de la présente invention peuvent être tous ceux déja connus en soi pour leur activité photoprotectrice. Ainsi, ils peuvent être notamment choisis parmi les oxydes de titane, de zinc, de fer, de zirconium, de cérium ou leurs mélanges.

De tels nanopigments d'oxydes métalliques, enrobés ou non enrobés, sont des produits connus de l'homme de l'art et sont en particulier décrits dans la demande de brevet EP-A- 0 518 773, dont l'enseignement est, à cet égard, inclus à titre de référence dans la présente description. A titre de nanopigments commerciaux complémentaires non cités dans la demande précitée mais également utilisables dans le cadre de la présente invention, on peut en outre mentionner les produits vendus sous les noms de marque UVT M 160, UVT M 212 et UVT M 262 par la Société KEMIRA, et MT 100 SA et MT 100 SAS par la Société TAYCA.

Comme indiqué précédemment, la taille moyenne des particules primaires des nanopigments présents dans les compositions selon l'invention est généralement comprise entre 5 nm et 100 nm, de préférence entre 10 et 50 nm.

Selon un mode préféré de réalisation des compositions antisolaires selon l'invention, on fait appel à des nanopigments minéraux à base d'oxyde de titane. Cet oxyde de titane peut se présenter sous une forme cristallisée de type rutile et/ou anatase, et/ou sous une forme amorphe ou substantiellemnt amorphe. Comme indiqué précédemment, ce pigment peut être alors enrobé ou non enrobé, mais de préférence on utilise des pigments enrobés, par exemple par de l'alumine et/ou du stéarate d'aluminium.

Les nanopigments sont généralement présents dans les compositions selon l'invention à une concentration comprise entre 0,1 et 30 % en poids, et de préférence entre 1 et 20 % en poids, par rapport au poids total de la composition.

D'un point de vue pratique, le système filtrant précité et les nanopigments ci-dessus sont bien entendu de préférence présents dans la composition finale dans des proportions respectives choisies de manière telle que l'effet de synergie, au niveau de l'indice de protection conféré par l'association résultante, soit optimal. La plage exacte des rapports pondéraux [système filtrant/nanopigments] dans laquelle cet effet de synergie optimal est effectivement atteint peut varier légèrement selon la quantité totale de filtres mise en oeuvre.

En outre, et d'une manière générale, on notera que les concentrations et rapports en filtres et nanopigments sont choisis de manière telle que l'indice de protection solaire de la composition finale soit de préférence d'au moins 2.

Enfin, toujours selon un mode préféré de réalisation de la présente invention, le support cosmétiquement acceptable dans lequel se trouvent contenus le système filtrant et les nanopigments est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants et notamment les antioxydants ARL, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, le ou les (co)émulsionnant(s) de 0,5 à 20 % en poids, de préférence de 2 à 10% en poids, par rapport à l'ensemble de la formulation, et les nanopigments de 0,1 à 30 % en poids, de préférence de 1 à 20% en poids, toujours par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLE 1

On a préparé une formulation antisolaire conforme à l'invention (Formulation 1) se présentant sous la forme d'une émulsion de type huile-dans-eau et contenant (les quantités sont exprimées en % poids par rapport au poids total de la composition) :

| | |
|---|---|
| - 2,4,6-tris[p-(2'-éthylhexyl-1'-oxycarbonyl)anilino]-1,3,5-triazine ("UVINUL T150")(premier filtre solaire) | 5 % |
| - Acide benzène 1,4-di(3-méthylidène-10-camphosulfonique) (deuxième filtre solaire) | 3 % MA |
| - Nanopigments de TiO₂ ("MT 100 T" de chez TAYCA) (taille moyenne des particules primaires : 15 nm) | 5 % |
| - Mélange monostéarate de glycérol/stéarate de polyéthylèneglycol à 100 moles d'OE ("Arlacel 165" d'ICI) | 2 % |
| - Acide stéarique | 2 % |
| - Alcool stéarylique | 1% |
| - Vaseline | 3% |
| - Polydiméthylsiloxane ("Silbione huile 70 047 V 300" de chez RHÔNE-POULENC) | 1 % |
| - Di-isooctanoate de Néopentyl Glycol ("Salacos 525" de chez SACI) | 23 % |
| - Glycérine | 5% |
| - Copolymère réticulé acide acrylique/acrylate d'éthyle en dispersion aqueuse à 28 % ("Acrysol 33" de ROHM & HAAS) | 1 % MA |
| - Triéthanolamine | qs pH 7 |
| - Conservateurs | qs |
| - Eau | qsp 100 % |

à laquelle on a en outre fait correspondre deux formulations comparatives de même composition que celle ci-dessus à ces différences près que la première formulation comparative (Formulation 2) était exempte de tout filtre solaire et ne contenait que les nanopigments de TiO₂ à la concentration de 5 %, et que la deuxième formulation comparative (Formulation 3) était exempte de nanopigments de TiO₂ et ne contenait que les premier et second filtres solaires précités, à raison, respectivement, de 5% et de 3%.

On a réalisé chacune de ces émulsions en dissolvant le premier filtre dans la phase grasse, puis en ajoutant les émulsionnants dans cette phase grasse portée aux environs de 80°C, et enfin en additionnant sous agitation rapide la phase aqueuse contenant le deuxième titre et préalablement chauffée à cette même température.

Pour chacune des formulations ainsi préparées, on a ensuite déterminé le facteur de protection solaire (SPF) qui leur était attaché. Celui-ci a été déterminé en utilisant la méthode *in vitro* décrite par B.L. DIFFEY et al. dans J. Soc. Cosmet. Chem. 40-127-133 (1989) ; cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.

Les résultats en facteur de protection moyen obtenus sont rassemblés dans le tableau I donné ci-dessous.

**Tableau I**

| | Formulations | | |
|---|---|---|---|
| | 1 (invention) | 2 (comparative) | 3 (comparative) |
| Premier filtre + Deuxième filtre (%) | 8 (5+3) | 0 | 8 (5+3) |
| Nanopigments (%) | 5 | 5 | 0 |
| SPF moyen (écart-type) | **27,4** (3,6) | **4,0** (0,2) | **12,3** (2,4) |

Ces résultats démontrent clairement l'effet de synergie obtenu avec la formulation 1 conforme à l'invention, le facteur de protection solaire attaché à cette dernière étant notablement et significativement supérieur à la simple somme arithmétique des facteurs de protection solaire attachés aux deux formulations comparatives correspondantes.

Toujours à des fins comparatives, on a par ailleurs préparé trois autres formulations 1', 2' et 3' identiques, respectivement, aux formulations 1, 2 et 3 ci-dessus, à cette seule différence près que les nanopigments de TiO₂ ont été ici remplacés par un pigment classique de TiO₂ dont la taille moyenne de particules est de l'ordre de 0,2 microns ("Hombitan anatase FF Pharma" de chez SACHTLEBEN), et on a ensuite étudié, comme ci-avant, les facteurs de protection solaires attachés à ces formulations. Les résultats sont rassemblés dans le tableau Il ci-dessous.

**Tableau II**

| | Formulations | | |
|---|---|---|---|
| | 1' (comparatif) | 2' (comparatif) | 3' (comparatif) |
| Premier filtre + Deuxième filtre (%) | 8 (5+3) | 0 | 8 (5+3) |
| Pigments classiques (%) | 5 | 5 | - 0 |
| SPF moyen (écart-type) | **14,5** (3,5) | **2,8** (0,6) | **12,3** (2,4) |

A la différence du cas précédent, ces résultats ne permettent de mettre en évidence aucun effet de synergie.

### EXEMPLE 2

On donne ci-dessous un autre exemple concret de composition antisolaire conforme à l'invention se présentant sous la forme d'une émulsion de type huile-dans-eau.

| | |
|---|---|
| - 2,4,6-tris[p-(2'-éthylhexyl-1'-oxycarbonyl)anilino]-1,3,5-triazine ("UVINUL T150") | 3 g |
| - Acide benzène 1,4-di(3-méthylidène-10-camphosulfonique) | 1,5 g |
| - Nanopigments de TiO₂ ("UVT-M262" de chez KEMIRA) | 3 g |
| - p-méthoxycinnamate de 2-éthylhexyle ("PARSOL MCX" de chez Givaudan)(filtre solaire) | 2 g |
| - Mélange d'alcool cétylstéarylique et d'alcool cétylstéarylique oxyéthyléné à 33 moles d'oxyde d'éthylène ("SINNOWAX AO" de chez Henkel)(émulsionnant) | 7 g |
| - Mélange de mono- di et tristéarate de glycérol (co-émulsionnant) | 2 g |
| - Adipate de diisopropyle | 20 g |
| - Polydiméthysiloxane | 1,5 g |
| - Alcool cétylique | 1,5 g |
| - Conservateurs | qs |
| - Eau distillée | qsp 100 g |

Cette émulsion a été préparée comme à l'exemple 1.

## Revendications

1. Compositions cosmétiques à usage topique, en particulier pour la photoprotection de la peau et/ou des cheveux, caractérisées par le fait qu'elles comprennent, dans un support cosmétiquement acceptable, (i) un système organique filtrant constitué par l'association d'un premier filtre qui est la 2,4,6-tris[p-(2'-éthylhexyl-1'-oxycarbonyl)anmino]-1,3,5-triazine avec un deuxième filtre qui est l'acide benzène 1,4-di(3-méthylidène-10-camphosulfonique), ce deuxième filtre pouvant éventuellement être sous une forme partiellement ou totalement neutralisée, et (ii) des nanopigments minéraux à base d'oxydes métalliques.

2. Compositions selon la revendication 1, caractérisées par le fait que ledit premier filtre est présent à une teneur comprise entre 0,1 et 10 % en poids par rapport au poids total de la composition.

3. Compositions selon la revendication 2, caractérisées par le fait que ladite teneur est comprise entre 0,5 et 5 % en poids.

4. Compositions selon l'une quelconque des revendications précédentes, caractérisées par le fait que ledit second filtre est présent à une teneur comprise entre 0,2 et 15 % en poids par rapport au poids total de la composition.

5. Compositions selon la revendication 4, caractérisées par le fait que ladite teneur est comprise entre 0,5 et 10 % en poids.

6. Compositions selon l'une quelconque des revendications précédentes, caractérisées par le fait que lesdits nanopigments sont choisis parmi les oxydes de titane, de zinc, de fer, de zirconium, de cérium et leurs mélanges, enrobés ou non.

7. Compositions selon la revendication 6, caractérisées par le fait les nanopigments sont à base d'oxyde de titane, enrobé ou non enrobé.

8. Compositions selon la revendication 7, caractérisées par le fait l'oxyde de titane est sous forme rutile, anatase ou amorphe.

9. Compositions selon l'une quelconque des revendications précédentes, caractérisées par le fait que lesdits nanopigments sont présents à une teneur comprise entre 0,1 et 30 % en poids par rapport au poids total de la composition.

10. Compositions selon la revendication 9, caractérisées par le fait que ladite teneur est comprise entre 1 et 20 % en poids.

11. Compositions selon l'une quelconque des revendications précédentes, caractérisées par le fait que ledit deuxième filtre répond à la formule suivante : 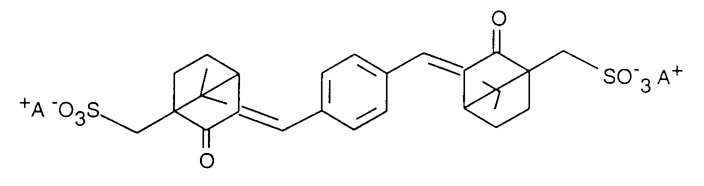 dans laquelle A désigne un atome d'hydrogène, un métal alcalin ou encore un radical NH(R)₃⁺ dans lequel les radicaux R, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺.

12. Compositions selon l'une quelconque des revendications précédentes, caractérisées par le fait que ledit support cosmétiquement acceptable se présente sous la forme d'une émulsion de type huile-dans-eau.

13. Compositions selon l'une quelconque des revendications précédentes, caractérisées par le fait qu'elles comprennent en outre un ou plusieurs filtres organiques complémentaires actifs dans l'UV-A et/ou UV-B, hydrophiles ou lipophiles.

14. Compositions selon la revendication 13, caractérisées par le fait que lesdits filtres organiques complémentaires sont choisis parmi les dérivés cinnamiques, les dérivés salicyliques, les dérivés du camphre, les dérivés de triazine, les dérivés de la benzophénone, les dérivés du dibenzoylméthane, les dérivés de β,β -diphénylacrylate, les dérivés de l'acide p-aminobenzoïque, les polymères filtres et silicones filtres

15. Compositions selon l'une quelconque des revendications précédentes, caractérisées par le fait qu'elles comprennent en outre au moins un agent de bronzage et/ou de brunissage artificiel de la peau.

16. Compositions selon l'une quelconque des revendications précédentes, caractérisées par le fait qu'elles comprennent en outre au moins un adjuvant choisi parmi les corps gras, les solvants organiques, les épaississants ioniques ou non ioniques, les adoucissants, les antioxydants et notamment les antioxydants ARL, les opacifiants, les stabilisants, les émollients, les silicones, les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

17. Compositions selon l'une quelconque des revendications précédentes, caractérisées par le fait qu'il s'agit de compositions protectices de l'épiderme humain ou de compositions antisolaires et qu'elles se présentent sous forme de dispersions vésiculaires non ioniques, émulsions, en particulier émulsions de type huile-dans-eau, crèmes, laits, gels, gels crèmes, suspensions, dispersions, poudres, bâtonnets solides, mousses ou spray.

18. Compositions selon l'une quelconque des revendications 1 à 16, caractérisées par le fait qu'il s'agit de compositions de maquillage des cils, des sourcils ou de la peau et qu'elles se présentent sous forme solide ou pâteuse, anhydre ou aqueuse, d'émulsions, de suspensions ou de dispersions.

19. Compositions selon l'une quelconque des revendications 1 à 16, caractérisées par le fait qu'il s'agit de compositions destinées à la protection des cheveux contre les rayons ultraviolets et qu'elles se présentent sous forme de shampooings, de lotions, de gels, d'émulsions, de dispersions vésiculaires non ioniques ou de laques pour cheveux.

20. Compositions selon l'une quelconque des revendications précédentes, caractérisées par le fait qu'elles présentent un indice de protection sur peau d'au moins 2.

21. Utilisation des compositions définies à l'une quelconque des revendications précédentes comme, ou pour la fabrication de, compositions cosmétiques pour la protection de la peau et/ou des cheveux contre le rayonnement ultraviolet, en particulier le rayonnement solaire.

22. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 20.

## Claims

1. Cosmetic compositions for topical use, in particular for sunscreening the skin and/or the hair, characterized in that they comprise, in a cosmetically acceptable support, (i) an organic screening system consisting of the combination of a first screening agent which is 2,4,6-tris[p-(2'-ethylhexyl-1'-oxycarbonyl)anilino]-1,3,5-triazine with a second screening agent which is benzene-1,4-di(3-methylidene-10-camphorsulphonic acid), it being possible for this second screening agent to be in a partially or totally neutralized form, and (ii) inorganic nanopigments based on metal oxides.

2. Compositions according to Claim 1, characterized in that the said first screening agent is present in a content of between 0.1 and 10% by weight relative to the total weight of the composition.

3. Compositions according to Claim 2, characterized in that the said content is between 0.5 and 5% by weight.

4. Compositions according to any one of the preceding claims, characterized in that the said second screening agent is present in a content of between 0.2 and 15% by weight relative to the total weight of the composition.

5. Compositions according to Claim 4, characterized in that the said content is between 0.5 and 10% by weight.

6. Compositions according to any one of the preceding claims, characterized in that the said nanopigments are chosen from titanium oxide, zinc oxide, iron oxide, zirconium oxide and cerium oxide and mixtures thereof, which may be coated or uncoated.

7. Compositions according to Claim 6, characterized in that the nanopigments are based on titanium oxide, which may be coated or uncoated.

8. Compositions according to Claim 7, characterized in that the titanium oxide is in rutile, anatase or amorphous form.

9. Compositions according to any one of the preceding claims, characterized in that the said nanopigments are present in a content of between 0.1 and 30% by weight relative to the total weight of the composition.

10. Compositions according to Claim 9, characterized in that the said content is between 1 and 20% by weight.

11. Compositions according to any one of the preceding claims, characterized in that the said second screening agent corresponds to the following formula 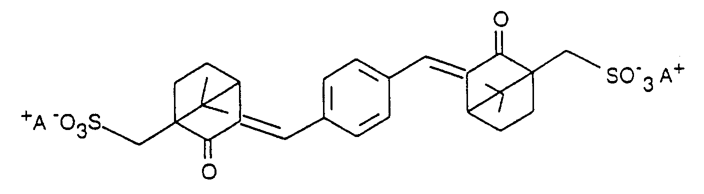 in which A denotes a hydrogen atom, an alkali metal or a radical NH(R)₃⁺ in which the radicals R, which can be identical or different, denote a hydrogen atom, a C₁-C₄ alkyl or hydroxyalkyl radical or alternatively a group Mⁿ⁺/n, Mⁿ⁺ denoting a polyvalent metal cation in which n is equal to 2 or 3 or 4, Mⁿ⁺ preferably denoting a metal cation chosen from Ca²⁺, Zn²⁺, Mg²⁺, Ba²⁺, Al³⁺ and Zr⁴⁺.

12. Compositions according to any one of the preceding claims, characterized in that the said cosmetically acceptable support is in the form of an emulsion of oil-in-water type.

13. Compositions according to any one of the preceding claims, characterized in that they also comprise one or more hydrophilic or lipophilic complementary organic screening agents active in the UV-A and/or UV-B range.

14. Compositions according to Claim 13, characterized in that the said complementary organic screening agents are chosen from cinnamic derivatives, salicylic derivatives, camphor derivatives, triazine derivatives, benzophenone derivatives, dibenzoylmethane derivatives, β,β-diphenylacrylate derivatives, p-aminobenzoic acid derivatives, screening polymers and screening silicones.

15. Compositions according to any one of the preceding claims, characterized in that they also comprise at least one agent for artificially tanning and/or browning the skin.

16. Compositions according to any one of the preceding claims, characterized in that they also comprise at least one adjuvant chosen from fatty substances, organic solvents, ionic or nonionic thickeners, softeners, antioxidants and in particular ARL antioxidants, opacifiers, stabilizers, emollients, silicones, α-hydroxy acids, antifoaming agents, moisturizers, vitamins, fragrances, preserving agents, surfactants, fillers, sequestering agents, polymers, propellants, basifying or acidifying agents and dyes.

17. Compositions according to any one of the preceding claims, characterized in that they are compositions for protecting the human epidermis or antisun compositions and in that they are in the form of nonionic vesicle dispersions, emulsions, in particular emulsions of oil-in-water type, creams, milks, gels, cream-gels, suspensions, dispersions, powders, solid tubes, mousses or sprays.

18. Compositions according to any one of Claims 1 to 16, characterized in that they are compositions for making up the eyelashes, the eyebrows or the skin and in that they are in solid or pasty, anhydrous or aqueous form or in the form of emulsions, suspensions or dispersions.

19. Compositions according to any one of Claims 1 to 16, characterized in that they are compositions intended for protecting the hair against ultraviolet rays and in that they are in the form of shampoos, lotions, gels, emulsions, nonionic vesicle dispersions or lacquers for the hair.

20. Compositions according to any one of the preceding claims, characterized in that they have a protection factor on the skin of at least 2.

21. Use of the compositions defined in any one of the preceding claims as, or for the manufacture of, cosmetic compositions for protecting the skin and/or the hair against ultraviolet radiation, in particular solar radiation.

22. Cosmetic treatment process for protecting the skin and/or the hair against ultraviolet radiation, in particular solar radiation, characterized in that it consists in applying an effective amount of a composition as defined in any one of Claims 1 to 20 to the skin and/or the hair.

## Patentansprüche

1. Kosmetische Zusammensetzungen zur topischen Anwendung, insbesondere zum Lichtschutz der Haut und/oder der Haare, dadurch gekennzeichnet, daß sie in einem kosmetisch akzeptablen Träger enthalten:
(i) ein organisches Filtersystem, das aus der Kombination eines ersten Filters, das 2,4,6-Tris[p-(2'-ethylhexyl-1'-oxycarbonyl)-anilino]-1,3,5-triazin ist, mit einem zweiten Filter besteht, das Benzol-1,4-di(3-methyliden-10-camphersulfonsäure) darstellt, wobei das zweite Filter gegebenenfalls in teilweise oder vollständig neutralisierter Form vorliegt,
und
(ii) anorganische Nanopigmente auf der Basis von Metalloxiden.

2. Zusammensetzungen nach Anspruch 1, dadurch gekennzeichnet, daß das erste Filter in einem Mengenanteil von 0,1 bis 10 Gew.-%, bezogen auf das Gesamtgewicht der Zusammensetzung, vorliegt.

3. Zusammensetzungen nach Anspruch 2, dadurch gekennzeichnet, daß der Mengenanteil 0,5 bis 5 Gew.-% beträgt.

4. Zusammensetzungen nach einem der vorhergehenden Ansprüche, dadurch gekennzeichnet, daß das zweite Filter in einem Mengenanteil von 0,2 bis 15 Gew.-%, bezogen auf das Gesamtgewicht der Zusammensetzung, vorliegt.

5. Zusammensetzungen nach Anspruch 4, dadurch gekennzeichnet, daß der Mengenanteil 0,5 bis 10 Gew.-% beträgt.

6. Zusammensetzungen nach einem der vorhergehenden Ansprüche, dadurch gekennzeichnet, daß die Nanopigmente unter Oxiden von Titan, Zink, Eisen, Zirconium und Cer sowie deren Gemischen ausgewählt und gegebenenfalls ummantelt sind.

7. Zusammensetzungen nach Anspruch 6, dadurch gekennzeichnet, daß es sich um Nanopigmente auf der Basis von Titanoxid handelt, die gegebenenfalls ummantelt sind.

8. Zusammensetzungen nach Anspruch 7, dadurch gekennzeichnet, daß das Titanoxid in Form von Rutil oder Anatas oder in amorpher Form vorliegt.

9. Zusammensetzungen nach einem der vorhergehenden Ansprüche, dadurch gekennzeichnet, daß die Nanopigmente in einem Mengenanteil von 0,1 bis 30 Gew.-%, bezogen auf das Gesamtgewicht der Zusammensetzung, vorliegen.

10. Zusammensetzungen nach Anspruch 9, dadurch gekennzeichnet, daß der Mengenanteil 1 bis 20 Gew.-% beträgt.

11. Zusammensetzungen nach einem der vorhergehenden Ansprüche, dadurch gekennzeichnet, daß das zweite Filter die Formel 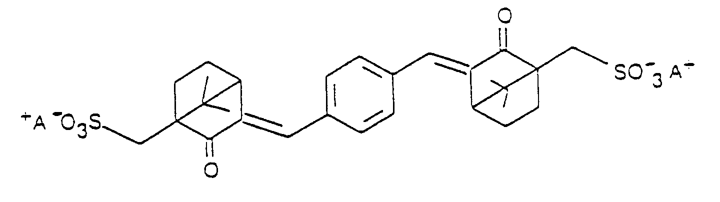 aufweist, worin A ein Wasserstoffatom, ein Alkalimetall oder eine Gruppe NH(R)₃⁺ bedeutet, in der die Substituenten R, die gleich oder verschieden sind, ein Wasserstoffatom, C₁₋₄-Alkyl, C₁₋₄-Hydroxyalkyl oder eine ionische Gruppe Mⁿ⁺/n bedeuten, wobei Mⁿ⁺ ein mehrwertiges Metallkation, bei dem n gleich 2, 3 oder 4 ist, und vorzugsweise ein Kation darstellt, das unter Ca²⁺, Zn²⁺, Mg²⁺, Ba²⁺, Al³⁺ und Zr⁴⁺ ausgewählt ist.

12. Zusammensetzungen nach einem der vorhergehenden Ansprüche, dadurch gekennzeichnet, daß der kosmetisch akzeptable Träger in Form einer Emulsion vom Wasser-in-Öl-Typ vorliegt.

13. Zusammensetzungen nach einem der vorhergehenden Ansprüche, dadurch gekennzeichnet, daß sie ferner ein oder mehrere zusätzliche hydrophile oder lipophile organische Filter enthalten, die im UV-A-Bereich und/oder im UV-B-Bereich wirksam sind.

14. Zusammensetzungen nach Anspruch 13, dadurch gekennzeichnet, daß die zusätzlichen organischen Filter ausgewählt sind unter Zimtsäurederivaten, Salicylsäurederivaten, Campherderivaten, Triazinderivaten, Benzophenonderivaten, Dibenzoylmethanderivaten, β,β-Diphenylacrylat-Derivaten, Derivaten der p-Aminobenzoesäure, polymeren Filtern und Siliconfiltern.

15. Zusammensetzungen nach einem der vorhergehenden Ansprüche, dadurch gekennzeichnet, daß sie ferner mindestens ein Mittel zur künstlichen Bräunung und/oder Braunfärbung der Haut enthalten.

16. Zusammensetzungen nach einem der vorhergehenden Ansprüche, dadurch gekennzeichnet, daß sie ferner mindestens einen Hilfsstoff enthalten, der ausgewählt ist unter Fettstoffen, organischen Lösungsmitteln, ionischen oder nichtionischen Verdickungsmitteln, Mitteln für die Geschmeidigkeit, Antioxidationsmitteln und insbesondere ARL-Antioxidationsmitteln, Trübungsmitteln, Stabilisierungsmitteln, Emollientien, Siliconen, α-Hydroxysäuren, Antischaummitteln, Hydratisierungsmitteln, Vitaminen, Parfums, Konservierungsmitteln, grenzflächenaktiven Mitteln, Füllstoffen, Maskierungsmitteln, Polymeren, Treibmitteln, Mitteln zum Alkalischmachen, Ansäuerungsmitteln und Färbemitteln.

17. Zusammensetzungen nach einem der vorhergehenden Ansprüche, dadurch gekennzeichnet, daß es sich um Zusammensetzungen zum Schutz der menschlichen Epidermis oder Zusammensetzungen zum Sonnenschutz handelt und sie in Form von nichtionischen Vesikeldispersionen, Emulsionen, insbesondere Emulsionen von Öl-in-Wasser-Typ, Cremen, Milchen, Gelen, Gelcremen, Suspensionen, Dispersionen, Pudern, festen Stiften oder Schäumen oder Sprays vorliegen.

18. Zusammensetzungen nach einem der Ansprüche 1 bis 16, dadurch gekennzeichnet, daß es sich um Zusammensetzungen zum Schminken der Wimpern, der Augenbrauen oder der Haut handelt und sie in fester oder pastoser, wasserfreier oder wasserhaltiger Form als Emulsionen, Suspensionen oder Dispersionen vorliegen.

19. Zusammensetzungen nach einem der Ansprüche 1 bis 16, dadurch gekennzeichnet, daß es sich um Zusammensetzungen zum Schutz der Haare gegen ultraviolette Strahlung handelt und sie in Form von Shampoos, Lotionen, Gelen, Emulsionen, nichtionischen Vesikeldispersionen oder Haarlacken vorliegen.

20. Zusammensetzungen nach einem der vorhergehenden Ansprüche, dadurch gekennzeichnet, daß sie einen Lichtschutzfaktor auf der Haut von mindestens 2 aufweisen.

21. Verwendung der Zusammensetzungen nach einem der vorhergehenden Ansprüche als kosmetische Zusammensetzungen oder zur Herstellung von kosmetischen Zusammensetzungen zum Schutz der Haut und/oder der Haare gegen ultraviolette Strahlung und insbesondere Sonnenstrahlung.

22. Verfahren zur kosmetischen Behandlung zum Schutz der Haut und/oder der Haare gegen Ultraviolettstrahlung, insbesondere Sonnenstrahlung, dadurch gekennzeichnet, daß es im Auftragen einer wirksamen Menge einer Zusammensetzung nach einem der Ansprüche 1 bis 20 auf die Haut und/oder die Haare besteht.
